# EUROPEAN PATENT SPECIFICATION

(11) **EP 3 562 443 B1**
(45) Date of publication and mention of the grant of the patent: **07.04.2021**
(21) Application number: 17837999.6
(22) Date of filing: 29.12.2017
(51) Int. Cl.: A61F 2/68, A61F 5/01, F16F 9/53, F16D 57/00, A61H 3/00, A61H 1/02, B25J 9/00, B25J 9/20, A63B 22/00, A63B 21/008, G05G 9/047, A61F 2/54, E05F 5/10, F16F 9/14, A63B 21/00

(54) **ESOSKELETON EQUIPPED WITH ELECTRO-OR MAGNETO- RHEOLOGICAL FLUID TYPE SEMI-ACTIVE JOINTS"**
EXOSKELETT MIT GELENKEN MIT SEMI-AKTIVER ELEKTRO- ODER MAGNETORHEOLOGISCHER FLÜSSIGKEIT
EXOSQUELETTE ÉQUIPÉ D'ARTICULATIONS SEMI-ACTIVES DE TYPE FLUIDE ÉLECTRO OU MAGNÉTO-RHÉOLOGIQUE

(30) Priority: 30.12.2016 IT 201600132874
(43) Date of publication of application: 06.11.2019
(73) Proprietor: Signo Motus S.R.L., 98168 Messina (IT)
(72) Inventor: SCATTAREGGIA MARCHESE, Sandro, 98100 Messina (IT); GIORGIANNI, Paolo, 98100 Messina (IT)
(74) Representative: Randazzo, Luigi
(86) International application number: PCT/IT2017/050010
(87) International publication number: WO 2018/122886

(56) References cited:
- DE-A1- 10 319 406
- US-A1- 2004 054 423

## Description

### TECHNICAL FIELD

The present invention relates to the field of wearable robotic devices that physically interact with humans, and in particular refers to a wearable exoskeleton, in particular for the upper limb.

Furthermore, the invention refers to an electro - or magneto- rheological fluid type semi-active joint purposely conceived to be used to make the exoskeleton.

### BACKGROUND ART

As known, wearable exoskeletons are mechanical devices that work in parallel with one or more limbs of a user, assisting his/her movements (Pons J. 2008, "Wearable robots: biomechatronic exoskeletons", Wiley). They are designed to obtain proper kinematic "compliance", which consists in not limiting the degrees of human limb's movement and, at the same time, ensuring the non-excess of movement. Such characteristics can be obtained through passive or active joints connected to each other through links. They can enhance or improve user's performances, or they are used for applications in virtual reality and entertainment domains.

In relation to the application field, it is possible to distinguish between wearable, rehabilitative and wearable assistive type exoskeletons. In particular, rehabilitative-type exoskeletons are essentially used to allow patients to perform customized rehabilitation protocols in case of neurological and / or orthopedic injuries, such as those caused by stroke, parkinson or cerebral paralysis, which somehow reduce limbs' motion capabilities. In particular, through these devices, it is possible to provide patients with automated personalized therapies as an alternative to the classic physiotherapeutic methods in which the success of the treatment depends totally on the therapist's experience.

The assistive exoskeletons, also known as orthoses, are devices with limited bulk and weight, used in case of traumas to the neuro-musculoskeletal apparatus. Their function is, in general, to keep human joints immobilized after an injury. See, for example, for the lower limbs, Low K. 2011, "Robot-assisted gait rehabilitation: From exoskeletons to gait systems" in the Defense Science Research Conference and Expo (DSR), p. 1 -10. There are also exoskeletons designed to increase user's performances, mainly used by healthy people, without particular pathologies, in work contexts where a certain degree of physical support is required to facilitate particular tasks that would otherwise require excessive effort or incorrect postures from an ergonomic point of view.

All the three classes of exoskeletons can then also be used in the field of virtual reality, for educational or entertainment purposes.

There are active exoskeletons, such as the one described in US2007225620, equipped with actuators at the joints, with considerable weights and overall dimensions.

The US20080009771A1 instead, realizes remote centrecentres of rotation and remote actuation through cables and pulleys. Actuators with annular guides are used to make the axes of the human body's joints coinciding as much as possible with the axes of the exoskeleton joints.

Active exoskeletons can also be used with passive functionality, by operating the actuators only as brakes to limit certain movements; for example to prevent limb hyper-extensions, to support loads, or to simulate the presence of obstacles in case of interaction with virtual reality environments.

Since the active exoskeletons are quite heavy, if used only with passive functionality, (Dollar A. 2008, "Lower Extremity Exoskeletons and Active Orthoses: Challenges and State-of-the-Art", IEEE Transaction on Robotics, Volume 24, Issue 1 , Feb. 2008), for some applications that do not require the presence of actuators, passive exoskeletal devices can be used, and they only generate interaction forces or torques, for example through springs or counterweights, which do not require any external power supply.

Among the exoskeletons with exclusively passive joints, US8142370B2, possessing one degree of freedom is known; it is provided with a rotational joint comprising an electro-rheological fluid element, usable where a variable resistance is required, thanks to adjustable braking torques, making it a semi-active joint. In one embodiment, a rotational joint of the elbow or knee exoskeleton is made close to the anatomical centre of rotation, to regulate the limb's degree of flexion-extension. In particular, the exoskeleton facilitates the correction of the hyper-extension of the joint through the fluid present inside the rotational joint which, being of the electro-rheological type, is used as a damping element with variable rotational stiffness and, in relation to the specific phases required by the therapy, it varies its viscosity, therefore its degree of resistance to rotation, following the presence of an electric field.

Through US8142370B2 it is possible, by adjusting the viscosity of the fluid and the duration of the damping, to obtain customized physiotherapeutic treatments in relation to the level of injury to be recovered. Moreover, this device, as it does not require electric motors, is extremely light and suitable for being used in everyday activities. However, the rotational joint of US8142370B2 has electrodes that involve constructive complexity and precise regulation. Furthermore, the presence of rotating or sliding contacts causes wear of these components with consequent limitation of its useful life. Furthermore, US8142370B2 does not facilitate the construction of other passive joints for the other degrees of freedom that a complete exoskeleton of upper or lower limb requires.

US 2004/054423 A1 discloses a prosthetic joint system which has an inner cylinder disposed within an interior cavity of a housing, where the cylinder rotates around a center axis. A sensor system is attached to an appendage that is connected to the cylinder. A dampening system with a power source is linked to the sensor system, cylinder and housing to control dampening of cylinder rotation. An attachment connects the housing to a user.

### DISCLOSURE OF INVENTION

The object of the present invention is to provide an exoskeletal device comprising joints of the semi-active type, which is able to reduce the articular stresses deriving from the limb proper weight, facilitating, in particular, the physical work load and favoring physical exercises necessary for the recovery of motor skills, after neurological or orthopedic traumas.

A further object of the invention is to provide an exoskeletal device that has reduced constructive complexity compared to prior art devices.

A further object of the invention is to provide an exoskeletal device that, as a whole, has a small footprint compared to prior art devices.

A further object is to provide an exoskeletal device which, as a whole, presents a reduced weight compared to prior art devices.

A further object of the invention is to provide an exoskeletal device of the upper limb which requires minimal maintenance and has a maximum useful life.

A particular object object of the invention is to provide an exoskeletal device that is suitable both in a domestic and in a hospital environment.

Another particular object of the invention is to provide an exoskeletal device having a modular structure based on the overall degrees of freedom that can be supplied to the user.

A further particular object of the present invention is to provide an exoskeletal device that can be used for the upper limb.

A further particular object of the invention is to provide an electro - or magneto - rheological fluid type joint that can be used to make an exoskeleton, finalized to reach the aforesaid objects.

These and further objects are achieved by a mechanism, in particular an exoskeleton comprising at least two links connected by a joint, in which the joint is of an electro- or magneto-rheological fluid type and comprises:
- a first body and a second body, slidely coupled to one another,
- wherein the first body comprises a piston having a head and a stem,
- in which the second body comprises a chamber in which the piston engages, the chamber forming a closed circuit filled with an electro- or magneto- rheologicalfluid, the closed circuit having a first branch in which the piston slides, and a second branch in which at least one pair of electrodes facing each other are present, defining a meatus between them where the fluid is present;
- a power supply arranged to feed electrodes of the second body so as to create an electric or magnetic field within the meatus;
- the piston of the first body being configured to push the fluid within the meatus and between the electrodes each time a relative motion occurs between the first and second body;
- and the power supply being configured to modulate the electric or magnetic field of the electrodes of the second body in such a way as to vary the resistance to the passage of the fluid within the meatus when the relative motion occurs between the first and second body.

The characteristic of this mechanism is that the first body and the second body are installed rotatable to one another around a rotation axis, that the piston has the stem of curvilinear shape concentric to the centre of rotation and that the second body has the branch of the chamber in which the piston flows with a curvilinear shape concentric to the axis of rotation. Therefore, the invention discloses a mechanism according to appended claim 1.

In this way a "flow mode" rotational configuration is obtained, which allows, compared to the known art, to have a fluid flow moved by a pressure gradient induced by the circular movement of the piston in the curvilinear chamber, causing the passage of fluid between two walls. This allows the viscosity of the fluid in the meatus to be varied as desired, determining a variation of the resistance to flow through modulation of the electric or magnetic field, and therefore to vary the resistance to rotation of the joint, until reaching the desired strength.

The fact that the pairs of electrodes belong only to the second body, during operation, allows considerable constructive simplicity with respect to US8142370B2. In fact, since the electrodes of each pair are present one on the first and one on the second body, in the assembly stage they require precise calibration of the reciprocal position, and they also require the presence of sliding contacts between the first and the second body.

These sliding contacts are not necessary in the invention, since it is enough to feed the electrodes present on the second body, while the first body has got no electrodes and does not require electrical power. The absence of sliding contacts between the first and second body of the joint according to the invention, in addition to a greater constructive simplicity, guarantees a greater duration with a lower capacity of wear.

The pair of electrodes of the second body is advantageously formed by a first electrode and a second electrode each having a plurality of protrusions and recesses parallel and concentric to the axis and extending in a circumferential manner for a predetermined angle, the two electrodes intercalating one on the other in a comb, so that each circumferential protrusion of the first electrode intercalates with adjacent circumferential protrusions of the second electrode. In this way, the meatus has a labyrinth radial section and it extends along a circumference for the relative angle, multiplying, by the number of protrusions, the portion of fluid flowing into the meatus, subjected to the electric or magnetic field.

This solution allows a constructive facility of the pair of electrodes, to be installed in a branch of the second body, or to be made jointly to a respective portion of the second body.

In particular, the sliding rotation between the first body and the second body is obtained through rotational supports, such as rolling bearings, bushings, curved guides, incorporated in seats obtained in the first body and / or in the second body, which makes a rotation between the second body and the first body, providing substantially a circular guide with low friction.

The first and second bodies have advantageously an open annular shape. In this way, the annular shape allows the realization of the circular sliding of the first on the second body and, at the same time, the definition of a free internal space, and the open shape allows the introduction of a limb, so as to make the remote axis of the joint coincide with a rotation axis of the skeletal joint and being at the same time easy to wear. For example, the open annular shape of the first and second body allows the realization of a joint having an open bracelet, which favors the "donning / doffing" procedures, therefore the wearing of the exoskeleton, reaching easily the coincidence between the centrecentre of rotation and the axis of the forearm or of the arm, obtaining internal-external rotation movements of the shoulder or pronation-supination of the elbow in such a way to simplify the kinematic configuration.

In a possible embodiment it is provided an exoskeletal device for a shoulder joint, having three degrees of freedom, comprising a first, a second and a third rotational joint, of which at least one rotational joint is made with a joint as defined above with an electro or magneto-rheological fluid type, with respective rotation axes aligned with the anatomic centre of the shoulder joint, wherein the above said rotational joints are connected to each other through respective links.

In particular, all three rotational couples of the shoulder joint are made with the electro- or magneto- rheological fluid type joint as defined above. In this way, an exoskeletal device is obtained with an electro- or magneto- rheological fluid type, realizing as much as possible a spherical kinematics of the shoulder joint, which excludes kinematic singularities within the workspace, and with the capacity to provide the user who wears it with abduction-abduction movements, flexion-extension, internal-external rotation of the shoulder articulation, of semi-active type, with at least one degree of freedom, or all three degrees of freedom of semi-active type controlled with electro- or magneto-rheological fluid.

The exoskeletal device for shoulder joint preferably comprises:
- a first shoulder rotational joint, to provide a shoulder abduction-abduction movement, having a first body integral with the user's trunk through a first shoulder link, and a second body rotatable on the first body, in particular within a 100° angular excursion,
- a second shoulder rotational joint, for the flexion-extension movement of the shoulder, having a first body integral with the second body of the first rotational joint through a second shoulder link, and a second body rotatable on the first body, in particular within a 180° angular excursion,
- and a third shoulder rotational joint for the internal-external rotation, or lateral medial rotation, having a first body integral with the second body of the second shoulder rotational joint through a third shoulder link, and a second body rotatable on the first body, in particular within a 180° angular excursion.

In particular, the third shoulder rotational joint is made with the first and second bodies with an open annular shape to allow easy insertion and extraction of the arm and to maintain the axis of the joint coincident with the axis of the arm.

Preferably, the adduction-abduction movement performed by the first shoulder rotational joint is delimited within an angular excursion of 100°, the flexion-extension movement performed by the second shoulder rotational joint is delimited within an angular excursion of 180° and the internal-external rotation, or lateral medial rotation, produced by the third shoulder rotational joint is delimited within a 180° angular excursion.

In a further embodiment it is foreseen an exoskeletal device for a two degree of freedom elbow joint, comprising rotational joints, with respective rotation axes coincident in the same centre of rotation in the centre of the elbow joint, in which at least one, or both of the above said elbow rotational joint are electro- or magneto-rheological fluid type, connected to each other through links.

In this way, as in the previous case, an exoskeletal device is obtained realizing a kinematics with two degrees of freedom of the elbow joint, which excludes kinematical singularities within the workspace, and it is able to provide the subject wearing it with flexion-extension and pronation-supination movements of the elbow joint.

Preferably, the above said exoskeletal device for an elbow joint comprises:
- a first elbow rotational joint, for the flexion-extension movement of the elbow, in particular having a first body integral with the forearm through a first elbow link and a second body rotatable on the first body,
- and a second elbow rotational joint for the pronation-supination movement, having a first body integral with the second body of the first elbow rotational joint through a second elbow link, and a second body rotatable on the first body.

In particular, the second elbow rotational joint is made with the first and second bodies with an open annular shape. Even in this case, the use of an open shaped joint, wearable around the forearm, allows the pronation-supination movement of the elbow joint, favors the kinematic simplicity of the exoskeletal device, reduces the overall dimensions and further facilitates the operations of "donning / doffing".

Preferably, the flexion-extension movement made by the first elbow rotational joint is delimited within an angular excursion of 140° and the pronation-supination movement, carried out by the second elbow rotational joint, is delimited within an angular excursion of 180°.

In particular, the pronation-supination movement is preferably carried out by means of a third handle-shaped elbow link connected to the second elbow rotational joint and adapted to be grasped by the user so as to establish a kinematic chain in such a way as to have correspondence between rotation of the second elbow rotational joint and the pronation-supination movement of the wrist joint.

In a further embodiment, the third elbow link can be replaced by a band, wrapping the wrist or the palm of the hand and connected to the second rotational joint, so as to relieve the user from gripping the handle, releasing the hand and making it free to perform additional tasks such as object grasping tasks or manipulation tasks.

In a further embodiment it is foreseen an exoskeletal device for the arm with five degrees of freedom, with at least one, and preferably all the rotational joint constituted by electro- or magneto-rheological fluid type joints, in which the kinematic sequence is obtained by coupling in series of the two shoulder and elbow exoskeletons, with the first body of the first elbow rotational joint integral with the second body of the third shoulder rotational joint through the first elbow link.

In this way, an exoskeletal device is obtained that achieves a five-degree kinematics of the shoulder and elbow joints, such as to exclude kinematic singularities within the working space, and is able to provide the subject wearing it with combined movements of shoulder and elbow joints.

This makes it possible to use this exoskeletal device to support a complete rehabilitation of the upper limb by placing angular constraints to prevent joint overextensions, offering a variable resistance to the motion independently on each specific joint and favoring the support of loads, by blocking the rotation of specific joints.

Preferably, the electro-rheological fluid type joint embeds a temperature sensor suitable for monitoring the temperature variations of the fluid contained in the chamber.

This allows, by constantly monitoring the temperature parameter, to optimize the performance of the device, since the properties of the fluid vary according to the temperature itself. The control of the temperature trend avoids the occurrence of greater energy consumption required by the device and related safety problems that could arise for the user if the temperature exceeds values outside the optimal operating range of the fluid.

In particular, the electro-rheological joint device operates within a temperature range of -10 to 40°C.

In a possible application of the invention, a system of an upper limb exoskeletal device can be provided, in one of the above defined possible forms, further integrating electrodes arrays selected from shoulder electrodes, arm electrodes and forearm electrodes, or combination, configured to be placed directly in contact with the user's skin surface, in particular through a supporting elastic mesh. Electrode arrays can be configured to provide electrical impulses to specific peripheral nerves in order to stimulate muscle contraction and / or movement of individual joints and / or detect electrical impulses to determine the electrical surface potential developed by muscle districts referred to the cutaneous surfaces that the arrays cover.

In this way, the addition of electrostimulation or electromyography is particularly suitable to support patient's movement or to detect his/her intentions of motion. For example, the exoskeletal device can be implemented for both the upper limbs, or for separate parts of the shoulder and forearm-elbow, to provide electrical stimulation in order to enable / facilitate the movement of a specific joint, dampening any abnormal synergies or involuntary movements by means of electro- or magneto-rheological fluid type joints. Another example consists in detecting the electrical surface potential developed by the muscular districts involved during the movement, modulating consequently the resistive force through the electro- or magneto-rheological fluid type joints in certain trajectories of the movement according to the user's intentions of motion.

According to another aspect of the invention, an exoskeletal system comprising an upper limb exoskeletal device, in one of the above defined forms, associated with a virtual or augmented reality device for applications in the domains of rehabilitation, fitness, entertainment, etc. The augmented or virtual reality device may be chosen among a headband, a viewer, or other device configured to be worn or carried by a user; the augmented or virtual reality device is configured to receive from the exoskeletal device data related to position, speed and force of the exoskeleton and to interact with the user according to these data.

In particular, it is possible to foresee a double upper limb exoskeleton made with electro- or magneto-rheological fluid type joint associated with the virtual or augmented reality device.

In this way, the system can be used through different types of applications / games and therefore used as a haptic interface for game environment and / or entertainment and / or fitness, as well as work equipment to alleviate weights, in environments where lifting loads is repetitively necessary. In fact, the joints can be stiffened to maintain a certain position with the load lifted, allowing to discharge stresses deriving from the weight of the load directly on the exoskeletal structure, for example on a suit worn by the user. Through the optional virtual or augmented reality device, the user can realize, in real time, the actual aid received by the device and he/she can be advised on the best position of limbs and body to maximize the functionality of the device.

In a further application, the mechanism mentioned above can be used to realize one or more electro- or magneto-rheological fluid type joints connected to respective links for the realization of non-exoskeletal variable resistance rotational devices for applications in rehabilitation, entertainment and fitness. Such devices can be configured according to one of the following kinematic schemes:
- a rotational joint with 1 degree of freedom, for example used to make a stepper;
- two interconnected rotational joint with a rigid link in order to obtain a coincident rotation centre obtaining a 2 degrees of freedom hinge, for example usable to make a joystick;
- a spherical joint that can be made by means of three interconnected joints in order to obtain 3 degrees of freedom, usable for example to make a rowing machine.

### BRIEF DESCRIPTION OF DRAWINGS

Further characteristics and / or advantages of the present invention will become clearer with the following description of an embodiment thereof, given as a non-limiting example, with reference to the attached drawings in which:
- Fig. 1 schematically shows, in section view, an electro-rheological fluid type joint, according to the invention, comprising a first and a second body pivotally coupled to each other, a curvilinear piston associated with the first body, a chamber containing electrodes, a power supply;
- Fig. 2 schematically shows, in section, a further view of an electro-rheological fluid type joint according to the invention, in which a temperature sensor is further visible, in addition to the elements described in Fig. 1;
- Fig. 3 is a schematic perspective view of an electro-rheological fluid type joint, according to the invention, comprising low-friction rolling elements able to generate relative rotation between the first and the second body;
- Fig. 4 schematically shows a sectional view of an electro-rheological fluid type joint, according to the invention, in which rolling bearings are visible together with angular position sensors to determine the rotational position and speed of the joint during its operation;
- Figs. 5-6 show respectively a perspective view and a sectional view of an application of an electro-rheological fluid type joint, according to the invention, having an open annular shape;
- Fig. 7 shows a perspective view of an embodiment of a magneto-rheological fluid type joint;
- Fig. 8 shows a kinematic scheme with three degrees of freedom, comprising movable elements with rotational joints connected to each other by links, able to represent the movements of the shoulder for what concerns the implementation of a shoulder exoskeleton with electro- or magneto-rheological fluid type joints according to the invention;
- Figs. 9-10 show, in two perspective views, a shoulder exoskeleton with three degrees of freedom comprising electro- or magneto-rheological fluid type joints, according to the invention, connected to each other through links;
- Fig. 11 shows a kinematic scheme with two degrees of freedom, comprising movable elements with rotational joints connected to each other by links, able to represent the movements of the elbow for what concerns the implementation of an elbow exoskeleton with electro- or magneto-rheological fluid type joint according to the invention;
- Figs. 12-13 show, in two perspective views, an elbow exoskeleton with two degrees of freedom comprising electro- or magneto-rheological fluid type joints, according to the invention, connected to each other through links;
- Fig. 14 shows a kinematic scheme with five degrees of freedom, comprising movable elements with rotational joints connected to each other by links, able to represent the movements of the upper limb for what concerns the implementation of an elbow exoskeleton with electro- or magneto-rheological fluid type joints according to the invention;
- Fig. 15 shows a perspective view of an exoskeleton with five degrees of freedom worn on a user's arm, comprising electro- or magneto-rheological fluid type joints, according to the invention, connected to each other by means of links and in which the joint of the shoulder, having three degrees of freedom, it is obtained from the intersection of an adduction-abduction axis, a flexion-extension axis, an internal-external rotation axis, and the elbow joint, having two degrees of freedom, it is obtained from the intersection of a flexion-extension axis with a pronation-supination axis;
- Fig. 16 shows a further perspective view of the upper limb exoskeleton of Fig. 15;
- Figs. 17-18 show, in two perspective views, a further embodiment of a limb exoskeleton with five degrees of freedom in which the kinematic chain proceeds from the shoulder to the wrist and it ends with a band around the user's wrist;
- Figs. 19 and 20 show block diagrams of the system architecture relative to a generic electro-rheological (Fig. 19) or magneto-rheological (Fig. 20) fluid type joint according to the invention;
- Figs. 21-22 show flow diagrams illustrating the control logic of an exoskeleton with five degrees of freedom for the upper limb with electro-rheological (Fig. 21) or magneto-rheological (Fig. 22) fluid type joints;
- Figs. 23-24 show, in an embodiment, an upper limb exoskeleton with five degrees of freedom equipped with electrostimulation and / or electromyography functionalities;
- Fig. 25 shows, in a perspective view, an embodiment of an upper limb exoskeleton with five degrees of freedom realized with electro- or magneto-rheological fluid type joints integrated with a virtual reality system;
- Fig. 26 shows, in a perspective view, an embodiment of an upper limb exoskeleton with five degrees of freedom, realized with electro- or magneto-rheological fluid type joints, worn on both the right and left upper limbs integrating a virtual reality system also intended for specific fitness applications.

### BEST MODE FOR CARRYING OUT THE INVENTION

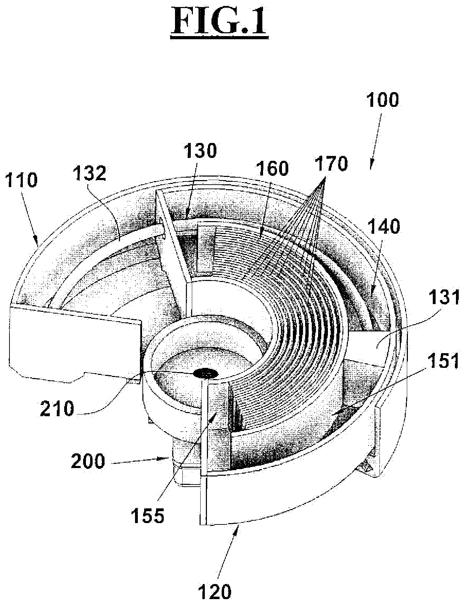

Regarding Fig. 1, according to what the present invention provides, an electro-rheological fluid joint 100 comprises a first body 110 and a second body 120, pivotally coupled to each other around a rotation centre 210 .

The first body 110 comprises a piston 130, formed by a head 131 and a shaft 132 of curvilinear shape concentric to the centre of rotation 210. The second body 120 comprises a chamber 140, also of curvilinear shape and concentric to the centre of rotation 210 .

The chamber 140 forms a closed circuit comprising two branches 151, 155 wherein, within the first branch 151, the piston 130 tightly slides, and in the second branch 155 there are at least one pair of electrodes 160 facing each other, defining between them meatus 170. Within the two branches 151, 155 of the circuit and within the meatus 170 between each pair of electrodes 160 there is an electro-rheological fluid.

The joint 100 further comprises a power supply 200, positioned externally to the body 120, and adapted to supply the electrodes 160 so as to create an electric field of adjustable intensity which passes through the meatus 170.

By creating a relative motion between the first body 110 and the second body 120, the fluid inside the chamber is moved by the piston 130 and it is forced to flow within the meatus 170 between the electrodes 160.

The power supply 200 is able, by supplying an operating power supply controlled by a program customized for the user, to energize the fluid present in the meatus 170, between the electrodes 160, and it varies its viscosity obtaining a resistance to the relative motion between the two bodies 110 and 120.

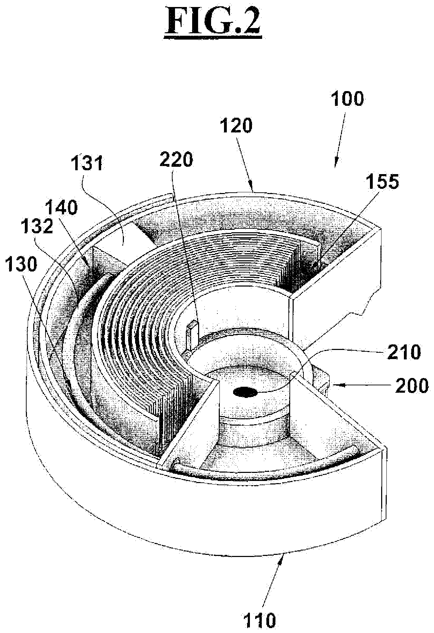

As shown in Fig. 2, the joint 100 may comprise within it a temperature sensor 220 adapted to provide indications about the temperature variations of the fluid contained in the joint 140, to verify that the temperature does not exceed a determined range of values.

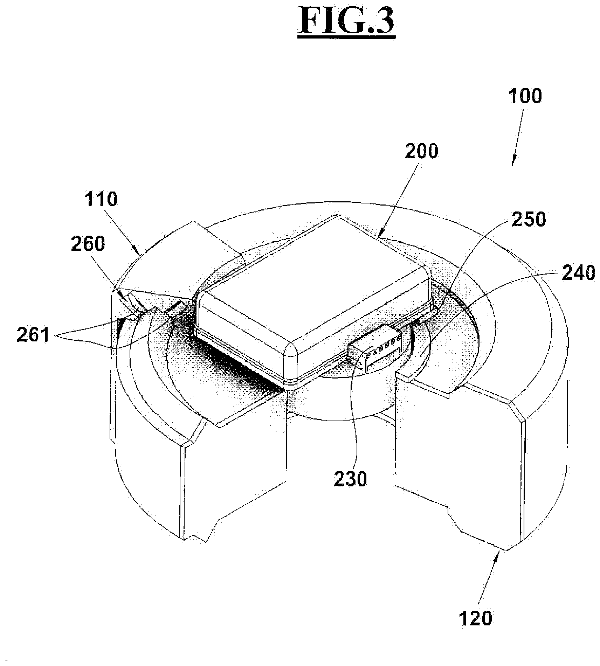

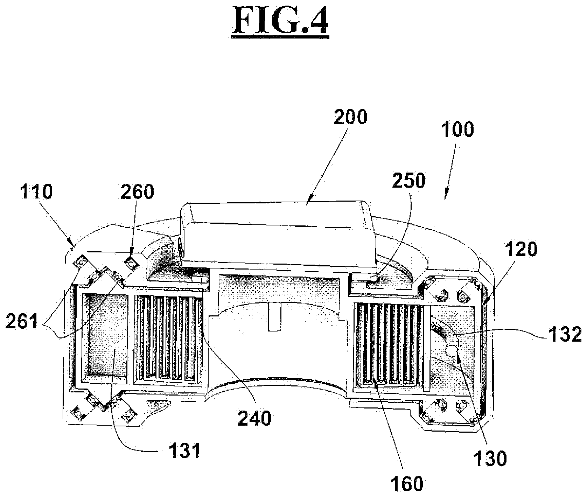

Regarding Fig. 3 and Fig. 4, in a possible embodiment, in order to facilitate the relative rotation between the two bodies 110 and 120, the bodies 110 and 120 incorporate seats 260 within which rolling bearings 261 are engaged, allowing therefore a low friction rotation between the two bodies. In particular, the interposition of rolling elements makes it possible to support the loads acting on the electro-rheological fluid type joint. Alternative low-friction elements of the known type between the two bodies can be easily foreseen by the person skilled in the art.

In a possible embodiment, shown in Fig. 3, the power supply 200 can incorporate a connector 230 for the communication of control data, via cable or wireless, to an externally positioned master control not shown in the figure.

Still as shown in Fig. 3 and in Fig. 4, in a possible embodiment, the angular position and velocity of the first body 110 with respect to the second body 120 can be detected by means of a read head 250 of position-speed with respect to a reading track 240, positioned on the second body 120.

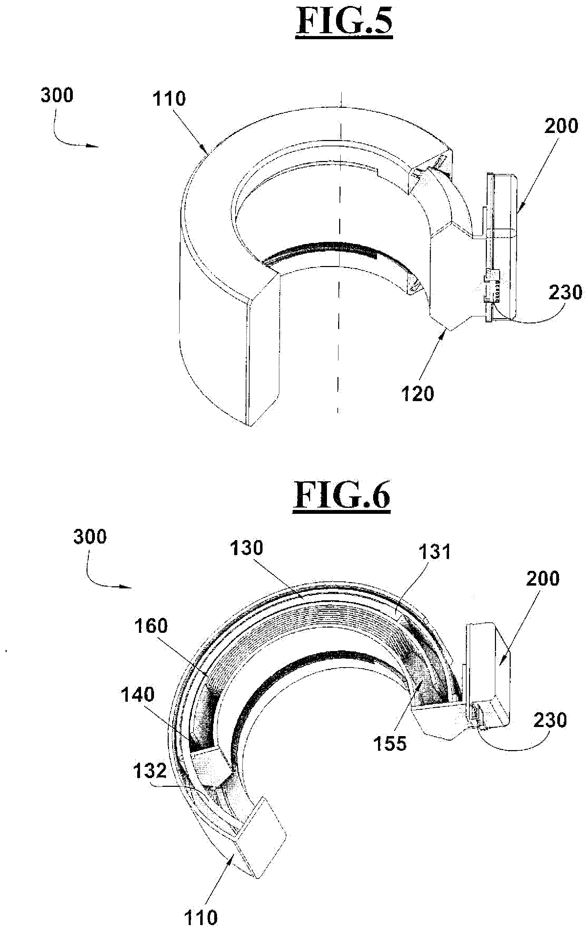

Figs. 5 and 6 show, respectively in a perspective view and in section, an embodiment of an electro-rheological fluid type joint 300 according to the invention having an open annular shape, so as to obtain a substantially hollow central area. The joint 300 has a chamber 140 of reduced thickness; in its first branch the head 131 of the piston 130, of curvilinear shape, slides. The presence of free internal space, and open form, as mentioned above, allow the introduction of a limb, so as to make the remote axis of rotation of the joint coincide with a rotation axis of the skeletal joint and have, at the same time, easy wearability.

The joint in the various embodiments, described above in detail, can be used to create mechanisms of various types, for example an exoskeletal type, as better described below, or not exoskeletal type.

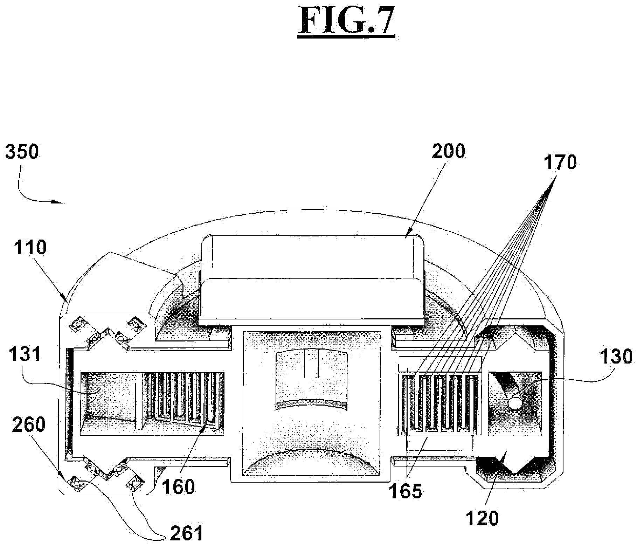

Regarding Fig. 7, a joint 350 is shown in a section perspective view, operating with magneto-rheological fluid. In this case, additional turns 165 are present which generate an additional tunable magnetic field in the inter-electrode region occupied by the pairs of electrodes 160 and by a magneto-rheological fluid present in the meatus 170. For the joint 350 described in the variant of Fig. 7 the embodiments described with reference to Figures 1-6 are applicable by a person skilled in the art, without the need for further detailed description.

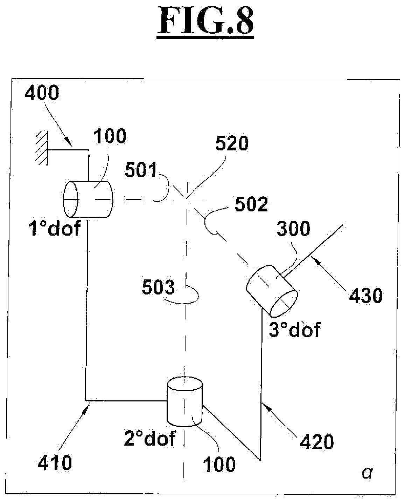

With the joints 100 or 300 or 350 above said, it is possible to obtain exoskeletons such as those illustrated, in their kinematic scheme, in Figs. 8-18.

In particular, Figs. 8-10 show a kinematic scheme with three degrees of freedom with rotational joints constituted by electro- or magneto-rheological fluid type joints, intended for the application of shoulder exoskeleton. In particular, the kinematic sequence comprises a first link 400 configured to be positioned in the dorsal part of a user's torso, and constrained in a way substantially integral with the bust itself or with a fixed support. Then a second link 410, connected to the first link 400 by means of a first rotational joint 100, analogous to the joint of Figs. 1-4 or Fig. 7, configured to have a first axis of rotation 501 substantially orthogonal to the front plane of the torso and passing through the centre of the shoulder joint 520. Then a third link 420, connected to the second link 410 through a second rotational joint 100, analogous to the joint of Figs. 1-4 or Fig. 7, configured to have a second axis of rotation 503 substantially orthogonal to the first axis of rotation 501 and incident with the first axis in the centre of the shoulder joint 520. Then, a fourth link 430, connected to the third link 420 through a third rotational joint 300, analogous to the joint of Figs. 5-6, configured to have a third axis of rotation 502 substantially orthogonal to the second axis of rotation 503 and incident with the first axis in the centre of the shoulder joint 520.

In this way, in a possible application form, a shoulder exoskeleton with three degrees of freedom is obtained, comprising three separate electro-rheological fluid types joints with rotation axes competing in the same point. In particular, through the first rotational joint 100 an abduction-adduction movement of the shoulder is obtained around a front plane passing through the user's shoulder joint. Through the second rotational joint 100 a flexion-extension movement of the shoulder is made around the second axis, substantially orthogonal to the first axis and parallel to the frontal plane, whose annular shape facilitates the "donning / doffing" operations and allows axial coincidence between exoskeleton and limb. Through the third rotational joint 300 an internal-external rotation movement of the shoulder is performed around a third axis, orthogonal to the first and second axis. Moreover, the axes of the three rotational joint converge in a single point 520, realizing as much as possible a spherical kinematics of the shoulder articulation which excludes kinematical singularities within the workspace.

Preferably, considering the adduction-abduction carried out by the shoulder rotational joint, the angular movement excursion is 100°, 180° in the case of flexion-extension and 180° for the internal-external rotation.

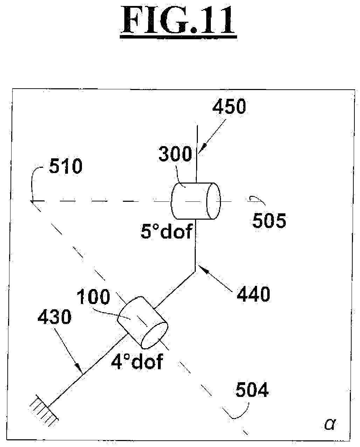

Figs. 11-13 illustrates an embodiment of a kinematic scheme with two degrees of freedom with rotational joint consisting of electro- or magneto-rheological fluid type joints, intended for the elbow exoskeleton application. In particular, the kinematic sequence is formed by a first link 430 configured to be positioned parallel to the forearm of a user. A second link 440, connected to the first link 430 through a first rotational joint 100, analogous to the joint of Figs. 1-4 or Fig. 7, configured to have a first axis of rotation 504 substantially orthogonal to the plane of flexion-extension of the elbow and passing through the centre of the elbow joint 510. Then, a third link 450, connected to the second link 440 through a second rotational joint 300, analogous to the joint of Figs. 5-6, configured to have a rotation axis 505 substantially orthogonal to the second axis of rotation 504 and passing through the centre of the elbow joint 510.

In this way, in a possible application form, an elbow exoskeleton with two degrees of freedom is obtained, comprising two separate electro- or magneto-rheological fluid type joints with rotation axes competing in the same point. In particular, through the first rotational joint 100 a flexion-extension movement is obtained around an axis 504 parallel to the front plane passing through the user's elbow joint. By means of the second rotational joint 300 a pronation-supination movement is performed around a second axis 505 substantially orthogonal to the first axis 504. Furthermore, the rotation axes converge in a single point 510, forming together a joint with two degrees of freedom for the elbow joint which excludes as much as possible kinematical singularities within the workspace.

Preferably, considering the flexion-extension movement performed by the elbow rotational joint, the angular excursion is 140° and, in case of the pronation-supination movement, it is 180°.

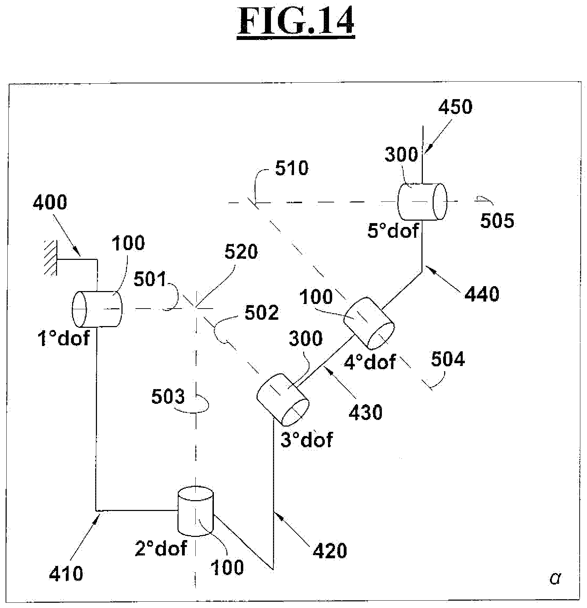

Figs. 14-16 show an embodiment of a kinematic scheme with five degrees of freedom with rotational joint constituted by electro- or magneto-rheological fluid type joints, intended for an exoskeleton application of the upper limb (shoulder-elbow), in which the kinematic sequence is formed by coupling in series the two kinematic chains of Fig. 8 and Fig. 11.

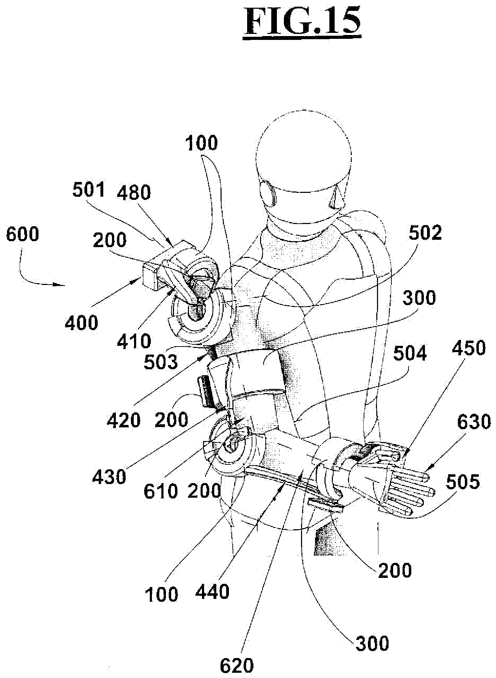

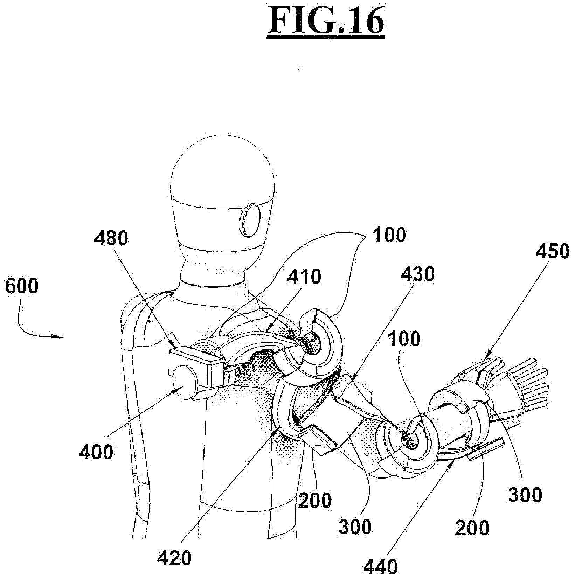

In more detail, with reference to Fig. 15 and Fig. 16, an anthropomorphic exoskeleton 600 with five degrees of freedom is shown for the upper limb worn by a user, having the kinematic scheme of Fig. 14. The exoskeletal device 600 is comprised of links 400, 410, 420, 430, 440, 450, connected to each other by means of rotational joints 100 and 300, and positioned along the arm 610, the forearm 620 and the user's hand 630.

In particular, link 400 can be used as a basic element to fix the exoskeletal device to the patient's limb, for example by using a harness (not shown), in case of using the exoskeleton in walking mode, or using a fixed support anchored firmly to the ground (not shown), in case of use in static conditions.

In both application contexts, wearable or anchored to the ground, through link 400 user's interaction forces with the exoskeleton are transferred to the harness or to the fixed support, so as not to burden the upper limb with the weight of the device.

Link 450 is a handle-shaped terminal element that serves, once held by hand 630, to transmit a force to the forearm. In this way, the exoskeleton can be used to support the weight of the upper limb during specific rehabilitation exercises or to provide a variable resistance to the movement of the limb through the modulation of the viscosity of the fluid present in the joints 100, 300.

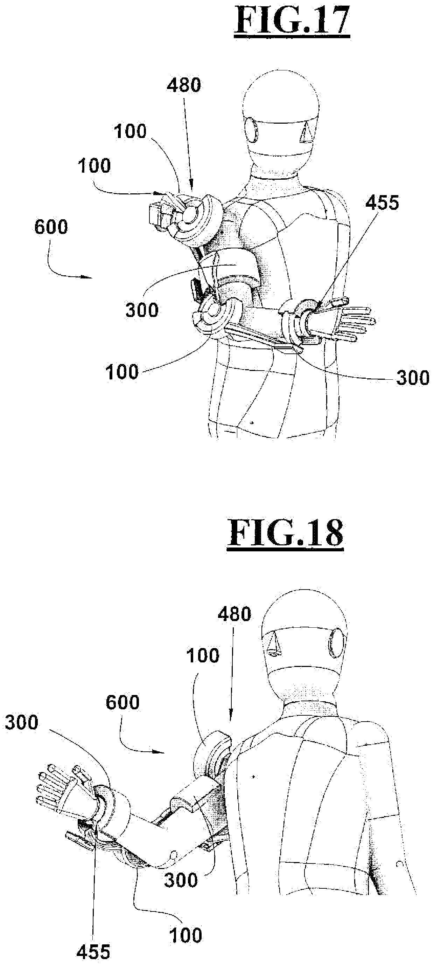

An alternative to link 450 is shown in Figs. 17 and 18, and it provides, instead of the handle 450, for a band 455, close to the wrist which serves to make the user's hand free. This band can also be tightened to the palm of the hand (not shown). In this way it is possible to grasp objects with the hand, for example in specific exercises for the user. The solution of Figs. 16 and 17 is similarly applicable to the exoskeleton for the elbow of Figs. 11-13.

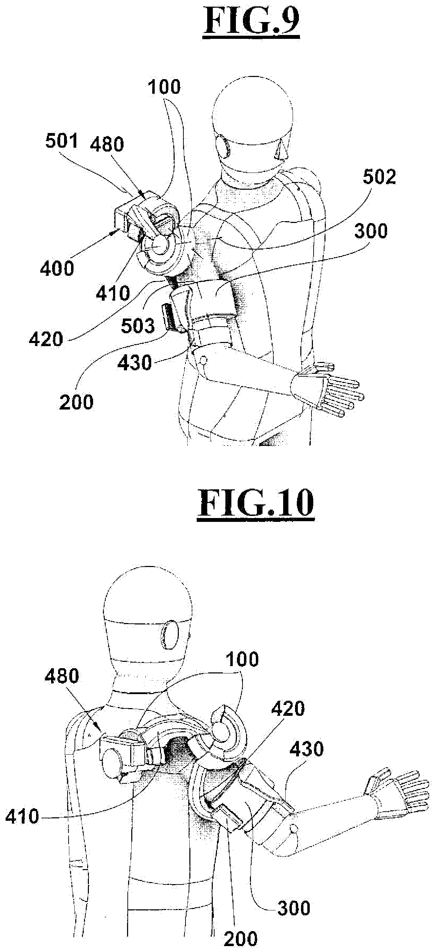

The electrical power to the power supplies 200, necessary for the operation of the joints, can be given by a control unit and power supply 480, visible in Fig. 9, 10, 15-18 and placed in the rear side of the user's shoulder between the link 400 and the joint 100.

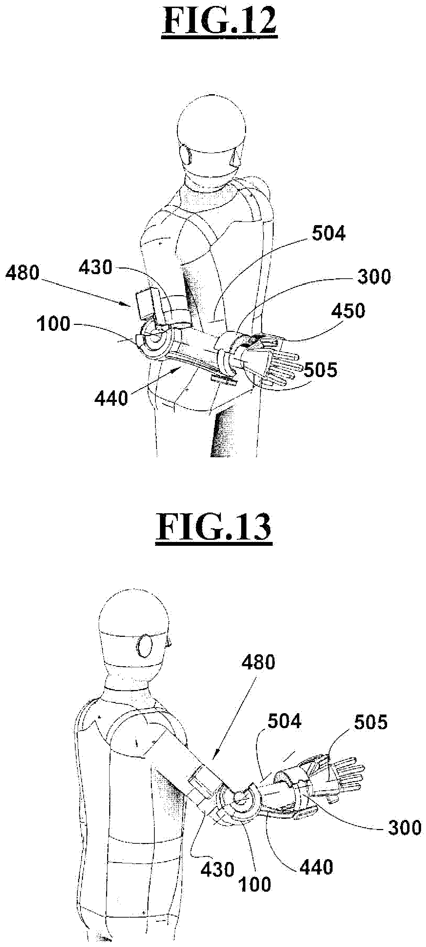

Instead, in the case of exoskeleton of the elbow of Fig. 12-13, the control and supply unit 480 can be placed integral with the link 430.

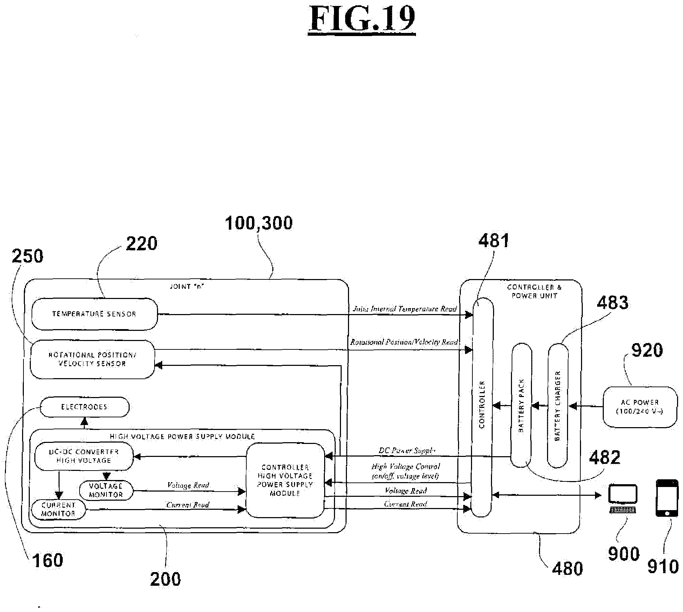

Fig. 19 shows a block diagram of the system architecture relating to a generic "n" joint, for example an electro-rheological fluid joint 100 or 300 as described above and a control and power supply unit, for example control unit and power supply 480 described above.

In particular, the control and power supply unit, like 480, includes:
- a controller 481, adapted to manage data, including fluid temperature data, position and angular velocity of the generic "n" joint, voltage and current of the supply module,
- a battery pack 482, designed to supply electrical power,
- and a recharging element 483 of the battery pack, adapted for example to be connected to a power supply 920.

The generic "n" joint, such as 100, 300 includes:
- a temperature sensor 220 suitable for monitoring the temperature variations of the fluid inside the joint chamber, capable of exchanging the temperature readings with the controller 481;
- a rotational sensor 250 to provide a reading of the position parameters and angular velocity of the joint, capable of exchanging the position and speed reading with the controller 481;
- electrodes 160;
- a voltage supply module 200 including a voltage supply control module, a high voltage converter, a voltage monitoring element, and a current monitoring element, capable of exchanging the respective readings with the controller 481.

Finally, a PC 900 or tablet / smartphone 910 manage the flow of data to the controller 481, according to specific training programs, assistance, virtual game, etc., implemented by a person skilled in the art according to user needs.

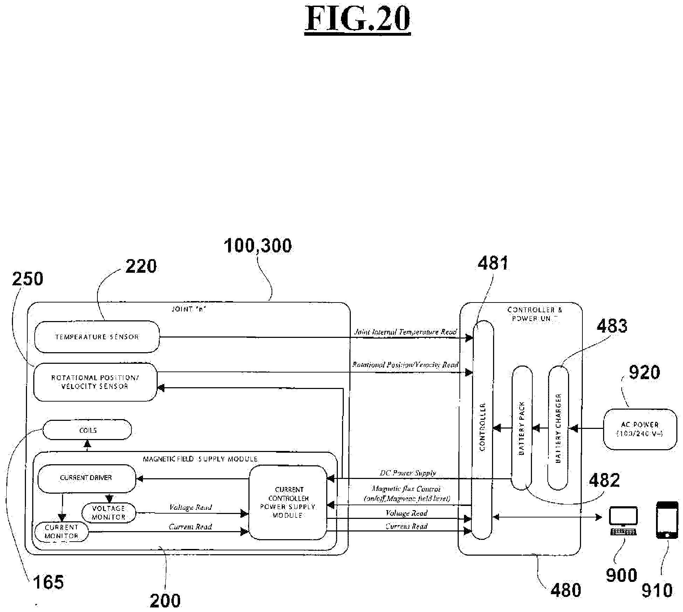

In Fig. 20, similarly, an example of a block diagram relating to a generic magneto-rheological joint is shown, such as for example the joint 350 of Fig. 7. The diagram is similar to that of Fig. 19, and therefore the relative description is not repeated, unlike the presence of the turns 165 in place of the electrodes 160.

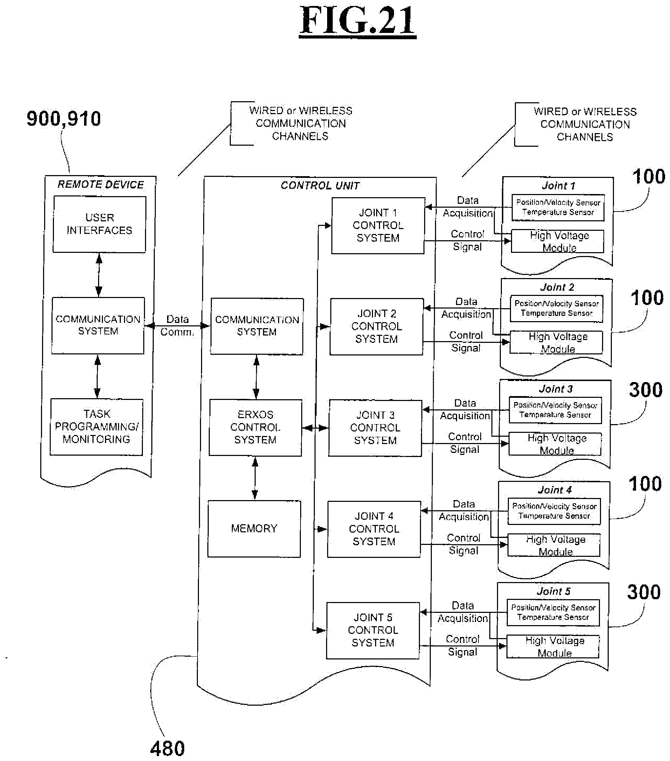

Fig. 21 shows an example of a block diagram related to the control flow of an exoskeletal device with five degrees of freedom for upper limb with electro-rheological joints, for example, as shown in Figs. 14-19. The remote device, such as PC 900 or tablet / smartphone 910, a control unit 480 and the five joints 100 or 300 communicate information such as temperature, speed of joints, position of joints and voltage of polarization of electrodes through channels of wired or wireless communication.

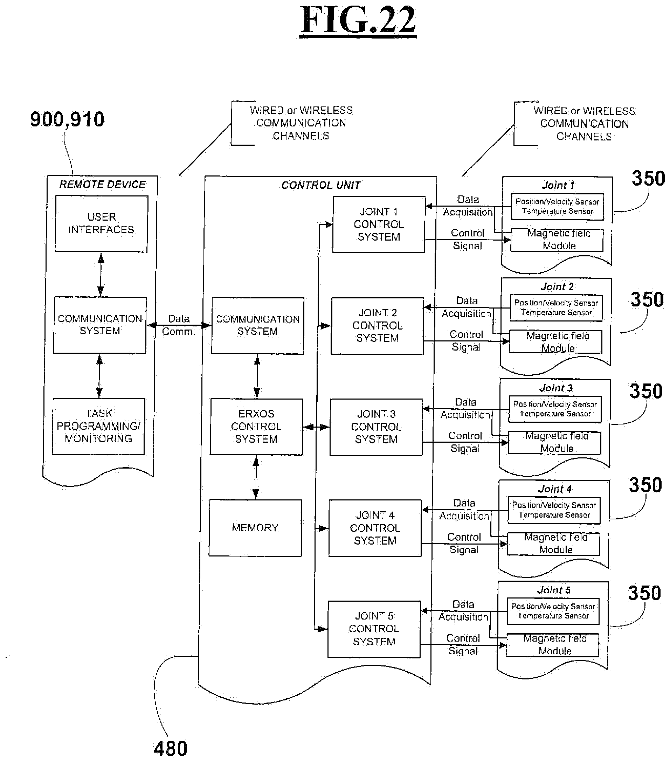

In a similar way to Fig. 21, in Fig. 22 it is shown, with the same numbering, an example of a block diagram related to the control flow of an exoskeletal device with five degrees of freedom for upper limb with magneto-rheological joints.

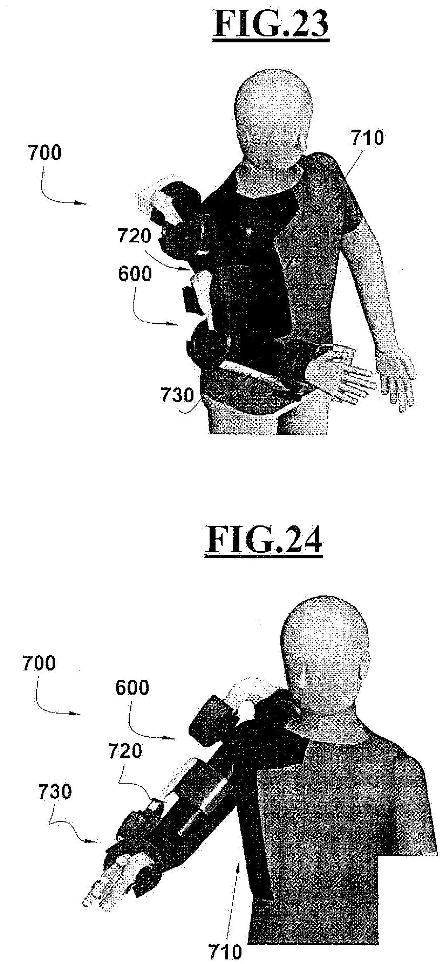

Figs. 23-24 show an embodiment of an exoskeletal-myoelectric system 700 formed by an exoskeletal device of the upper limb, such as that indicated for example with 600 in Fig. 14-16 with five degrees of freedom, further integrating 710 electrode array of shoulder, arm electrodes 720 and forearm 730 electrodes, directly in contact with the skin surface of the user, for example by means of a supporting elastic mesh.

Electrode arrays 710-730 can have the functionality of supplying electrical impulses to specific peripheral nerves, in order to stimulate the muscle contraction and / or the movement of the single joints. In this mode, the controller of the electrostimulation system is connected to the controller 481 of the exoskeleton 600 (Fig. 19-20) in order to perform muscle stimulation as a function of the rehabilitation exercise.

An application example may consist in the possibility of providing electrical stimulation to the muscles of the shoulder, arm or forearm, respectively through the electrode arrays 710, 720, 730 to enable / facilitate the movement of a specific articulation, and at the same time to correct any abnormal synergies or involuntary movements by means of the joints 100, 300, 350 with electro- or magneto-rheological fluids of the exoskeleton 600, providing angular constraints and damping to the respective five degrees of freedom of the shoulder and of the elbow.

In another example, it is possible to provide a selective electrical stimulation through the electrode arrays 730 necessary to open and / or close the user's hand when the upper limb is in a certain position, to perform combined rehabilitative exercises for the hand and the shoulder and elbow joints, through the joints 100, 300, 350 with electro- or magneto-rheological fluids of the exoskeleton 600.

Electrode arrays 710-730 can also have the function of detecting electrical impulses to determine the surface electric potential developed by the muscular district involved during movement. This detection can allow to selectively activate the resistance of the electro- or magneto-rheological joints in order to provide suitable resistance to the motion as a function of the movement, the position of the exoskeleton and the task to be performed. This modality allows the selective reinforcement of particular muscular districts by controlling the resistive force in certain trajectories of the movement according to the intentions of the user's motion.

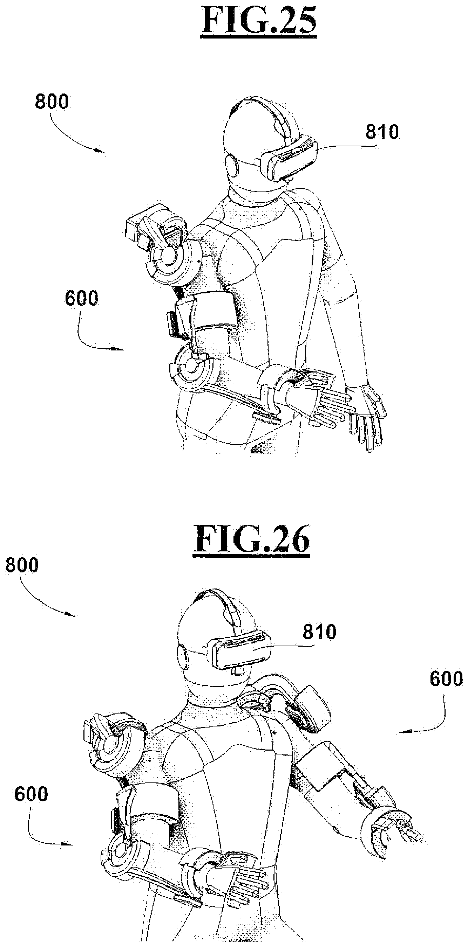

Fig. 25 shows an embodiment of an exoskeletal-visual system 800 formed by an exoskeletal device of the upper limb, such as that indicated for example with 600 in Fig. 14-18 with five degrees of freedom, and a device of virtual or augmented reality 810 for applications in rehabilitation, fitness, entertainment, etc. In this case, the user wearing the upper limb exoskeleton 600 and wearing the virtual or augmented reality device 810, such as a headset, a viewer, or other device connected for example to a PC or tablet / smartphone, can perform exercises in a virtual or augmented environment by defining suitable targets with different levels of difficulty.

In case of rehabilitation, combining the exoskeleton with gaming devices, virtual reality or augmented reality, it is possible to provide the patient with support regarding the progress of the rehabilitation treatment. For example, the analysis of position, velocity and force data of the exoskeleton during use provides information about the outcome of the therapeutic treatment.

Similarly, Fig. 26 show an embodiment of a system 800 made with a double exoskeleton 600 of an upper limb with five degrees of freedom obtained through electro- or magneto-rheological fluid type joints, possibly coupled to a virtual or augmented reality device 810. In this case, the application form can contemplate the use of the exoskeleton on both the right and left upper limbs cooperating during the execution of exercises for applications in rehabilitation, fitness, entertainment or sports domains. In this case, a virtual or augmented reality device 810 may present the user with indications for exercises useful for improving coordination of movements for an athletic training. During the exercises, the system is able to record data related to athletic performances, such as the energy spent, the scores acquired in relation to the objectives set. In this way, a highly personalized athletic training program is provided.

As an alternative to the virtual or augmented reality device 810, it is also possible that the system includes the association with an interface console with smart TVs, video game monitors, etc,

In the case of non-exoskeletal mechanisms, one or more electro- or magneto-rheological fluid joints, as described above, can be used in connection with the respective links for the realization of non-exoskeletal rotational devices with variable resistance, for applications in rehabilitation, entertainment and fitness. These can be configured as: a single joint realizing a rotational joint with one degree of freedom, for example a stepper; two joints forming two interconnected rotational joint with a rigid link, so as to obtain a coincident rotation centre obtaining a hinge with 2 degrees of freedom, for example to be used as a joystick; a spherical joint by means of three interconnected joints in order to obtain 3 degrees of freedom, for example to make a rowing machine. Such examples of one, two or three degrees of freedom mechanisms are not described in details, as they can be easily implemented by a person skilled in the art. In fact, in the case of the stepper, it is sufficient to use the joint as shown in Figs. 1-5 as a stepper hinge. In the case of the joystick, the two-degree freedom mechanism can be implemented in a similar manner to the elbow exoskeleton of Figs. 11-13 with non-exoskeletal configuration. In the case of the rowing machine, the three degrees of freedom mechanism can be implemented in a similar manner to the shoulder exoskeleton of Figs. 8-10 with non-exoskeletal configuration.

The above description of some specific embodiments is able to show the invention from the conceptual point of view so that others, using the known art, will be able to modify and / or adapt in various applications such specific embodiment without further researches and without departing from the scope of protection of the invention, as defined by the appended set of claims.

## Claims

1. A mechanism_comprising_at least two links (400-410; 410-420;420-430) -connected by a joint (100:100:300), wherein said joint (100;100; 300) is of electro- or magneto- rheological fluid type and comprises:
- a first body (110) and a second body (120), slidably coupled to each other,
- wherein the first body (110) comprises a piston (130) having a head (131) and a stem (132),
- wherein the second body comprises a chamber (140) in which the piston (130)slidingly engages,the chamber (140) arranged as a closed circuit filled with an electro- or magneto- rheological fluid, wherein said circuit has a first branch (151) of said chamber (140) in which said piston (130)tightly slides, and a second branch (155) of said chamber (140) in which at least one couple of electrodes (160) is provided that are facing each other, defining between said electrodes a channel (170) where said fluid is present;
- a supply unit (200) arranged to supply said electrodes (160) of said second body (120) to make an electric or magnetic field that passes through said channel (170);
- said piston (130) of said first body (110)configured for pushing said fluid in said channel (170) and to pass between said electrodes (160)when a relative movement between said first body (110)and second body (120) occurs;
- and said supply unit (200)configured for modulating said electric or magnetic field of the electrodes (160) of said second body (120)in order to adjust the resistance against the movement of said fluid in said channel (170) when said relative movement between said first (110)and second body (120)occurs;
- wherein said first body (110) and second body (120) are pivotally coupled to each other about a center of rotation (210), wherein said piston (130) has said stem (132) of curvilinear shape concentric to said center of rotation (210, and wherein said first branch (151) of said chamber (140) and said second branch (155) of said chamber (140) have curvilinear shape and are concentric to said center of rotation(210).

2. The mechanism according to claim 1, wherein said couple of electrodes (160) of said second body (120)comprises a first electrode and a second electrode having each a plurality of protrusions and recesses parallel and concentric to said axis and extending about a circumference for a predetermined angle, the two electrodes interdigitated on each other, so that each circumferential protrusion of the first electrode is interdigitated with adjacent circumferential protrusions of the second electrode.

3. The mechanism according to claim 1, wherein the first body (110)and a second body (120)are pivotally coupled to each other by bearings (261), in particular rolling bearings, incorporated in housings (260) obtained in the first body (110) and in the second body (120) and arranged to permit a low friction rotation between the second body (120) and the first body (110).

4. The mechanism according to claim 1, wherein said first body (110)and second body (120) have a ring-like open shape.

5. An exoskeleton mechanism according to claim 1, further disclosing three degrees of freedom for shoulder articulation, comprising a first, a second and a third rotational joint, of which at least one is a joint of electro- or magneto- rheological fluid type according to the joint of the mechanism of claim 1, with respective rotation axes concurrent in a same point coincident with the center of the shoulder articulation, wherein said rotational joints are connected to each other through respective links (400, 410, 420, 430)

6. The exoskeleton mechanism according to claim 5, further comprising:
- a first shoulder rotational joint (100), arranged to provide an adduction-abduction movement of the shoulder, having a first body (110) integral to the trunk of the user through a first shoulder link (400), and a second body (120) rotatable on the first body, in particular within an angular excursion of 100°;
- a second shoulder rotational joint (100), arranged to provide a flexion/extension movement of the shoulder, having a first body (110) integral to the second body (120) of the first rotational joint (100) through a second shoulder link (410), and a second body (120) rotatable on the first body (110), in particular within an angular excursion of 180°;
- a third shoulder rotational joint (300), arranged to provide an internal-external movementof the shoulder, or medial-lateral, having a first body (110) integral to the second body (120) of the second shoulder rotational joint (100) through a third shoulder link (420), and a second body (120) rotatable on the first body (110), in particular within an angular excursion of 180°,

7. The exoskeleton mechanism according to claim 6, wherein the third shoulder rotational joint (300)provides said first (110) and said second body (120) with a ring-like open shape.

8. An exoskeleton mechanism according to claim 1, further disclosing two degrees of freedom for elbow articulation, comprising two rotational elbow joints with respective rotation axes concurrent in a same center of rotation in the center of the elbow articulation, wherein at least one, or both, said rotational elbow joints are joints of electro- or magneto- rheological fluid type according to the joint of the mechanism of claim 1, connected to each other through links (430, 440, 450)

9. The exoskeleton mechanism according to claim 8, further comprising:
- a first elbow rotational joint (100), arranged to provide a flexion/extension movement of the elbow, in particular having the first body (110) configured to be integral to a forearm (620) through a first elbowlink (430), and a second body (120) rotatable on the first body (110)
- a second elbow rotational joint (300), arranged to provide a prono-supination movement_of the elbow, having a first body (110) integral to the second body (120) of the first rotational elbow articulation through a second elbow link (440), and a second body (120) rotatable on the first body (110)

10. The exoskeleton mechanism according to claim 9 , wherein the second rotational elbow joint (300)provides said first (110) and said second body (120) with a ring-like open shape.

11. The exoskeleton mechanism according to claim 9, wherein the flexion/extension movement of the first rotational elbow joint (100) is defined within an angular excursion of 140° and the movement of prono-supination of the second rotational elbow joint (300) is defined within an angular excursion of 180°.

12. An exoskeleton mechanism according to claim 1, further disclosing the features of being for a limb and disclosing five or more degrees of freedom, comprising rotational joints, of which at least one is a joint of electro- or magneto- rheological fluid type according to the joint of the mechanism of claim 1, obtained by combining in series an exoskeleton mechanism for shoulder articulation and an exoskeleton mechanism for elbow articulation according to claims 5 and 8 respectively, with the first body (110) of the first rotational elbow joint (100)integral to the second body (120)of the third rotational shoulder joint (300) through the first elbow link (430).

13. An exoskeleton mechanism for upper limb according to claim 8, wherein the movement of prono-supination is assisted by a third elbow link having a shape of a handle configured to be grasped by the user or of a belt configured to be wound about the wrist or the palm of the hand of a user, said third elbow link being connected to the second rotational elbow joint, said third elbow link arranged to establish a kinematical chain that is blocked to the user's limb and to provide correspondence between a rotation of the second rotational elbow joint and the movement of prono-supination of a user's wrist articulation.

14. An exoskeleton-mioelectric system (700) comprising an exoskeleton mechanism (600), according to any of claims 5, 8 or 12, and further comprising an array of electrodes selected from the group consisting of: shoulder electrodes (710), arm electrodes (720), and forearm electrodes (730), configured to be located directly in contact with the skin of the user, in particular by a resilient knitted support, said array of electrodes (710, 720, 730) configured to provide electric pulses to specific peripheral nerves, in order to stimulate muscle contraction and/or the movements of body articulations and/or to measure electric pulses for determining surface electric potential developed by muscular zones referred to said skin.

15. An exoskeleton system (800) comprising an exoskeleton mechanism (600) according to any of claims 5, 8 or 12, integrating a device for virtual or augmented reality (810) for applications in the fields of rehabilitation, fitness, entertainment, etc., said device for virtual or augmented reality (810) being selected from the group consisting of: a helmet, a headset, or other device configured for being worn or brought by a user, said device for virtual or augmented reality (810) configured for receiving from said exoskeleton device data of position, speed and force of the exoskeleton mechanism and to interact with said user responsive to said data, in particular a double exoskeleton (600) is provided equipped with joints of electro- or magneto- rheological fluid type and with said device for virtual or augmented reality (810).

## Patentansprüche

1. Ein Mechanismus mit mindestens zwei Gliedern(400-410;410-420;420-430) verbunden durch ein Gelenk (100;100;300), wobei die Verbindung ist vom Typ mit elektro- oder magnetorhetorologischer Flüssigkeit umfasst:
- einen ersten Körper (110) und einen zweiten Körper (120), die reibungslos aneinander befestigt sind,
- bei dem der erste Körper (110) einen Kolben (130) mit einem Kopf (131) und einer Kolbenstange (132) und einer Kolbenstange (131) umfasst,
- bei dem der zweite Körper enthält eine Kammer (140), in der der Kolben zum Gleiten in Eingriff ist (130), die Kammer einen geschlossenen Kreislauf bildet, der mit einem elektro- oder magnetorheologischen Fluid gefüllt ist, wobei der Kreislauf einen ersten Zweig (151) der Kammer (140), in dem der Kolben (130) dicht gleitet, und einen zweiten Zweig (155) der Kammer (140) aufweist, in dem mindestens ein Paar einander gegenüberliegender Elektroden (160) vorhanden ist, die zwischen den Elektroden einen Schmierspalt (170) definieren, in dem das Fluid vorhanden ist;
- eine Stromversorgungseinheit (200), die so angeordnet ist, dass sie die Elektroden (160) des zweiten Körpers (120) versorgt, um so ein elektrisches oder magnetisches Feld zu erzeugen, das durch den Schmierspalt (170) verläuft;
- dieser Kolben (130) des ersten Körpers (110) so konfiguriert ist, dass er das Fluid in dem Schmierspalt(170) drückt und somit jedes Mal, wenn eine Relativbewegung zwischen dem ersten Körper (110) und dem zweiten Körper (120) auftritt, zwischen den Elektroden (160) hindurchgeht;
- und wobei der Speiser (200) so konfiguriert ist, dass sie das elektrische oder magnetische Feld der Elektroden (160) des zweiten Körpers (120) derart moduliert, dass der Widerstand gegen den Durchgang der Flüssigkeit innerhalb des Schmierspaltes (170) variiert wird, wenn die relative Bewegung zwischen dem ersten (110) und dem zweiten Körper (120) auftritt;
- bei dem der erste Körper (110) und der zweite Körper (120) um ein Drehzentrum (210) drehbar aneinander befestigt sind, und bei dem der Kolben (130) und die Kolbenstange (132) in dem Drehzentrum (210) eine konzentrische gekrümmte Form aufweisen, und bei dem der erste Schenkel (151) der Kammer (140) und der zweite Schenkel (155) der Kammer (140) in dem Drehzentrum (210) eine konzentrische gekrümmte Form aufweisen.

2. Mechanismus nach Forderung 1, bei dem das Elektrodenpaar (160) des zweiten Körpers (120) durch eine erste Elektrode und eine zweite Elektrode gebildet wird, die jeweils eine Vielzahl von Vorsprüngen und Ausnehmungen aufweisen, die parallel und konzentrisch zu der Achse verlaufen und sich um den Umfang in einem vorbestimmten Winkel erstrecken, wobei die beiden Elektroden durch einen Kamm übereinander angeordnet sind, so dass jeder Umfangsvorsprung der ersten Elektrode in benachbarte Umfangsvorsprünge der zweiten Elektrode eingreift.

3. Mechanismus nach Forderung 1, bei dem der erste Körper (110) und der zweite Körper (120) mittels Lagern (261), insbesondere Wälzlagern, die in Gehäuse (260) eingebaut sind, die in den ersten Körper (110) und in den zweiten Körper (120) eingearbeitet sind und eine Rotation mit geringer Reibung zwischen dem zweiten Körper (120) und dem ersten Körper (110) realisieren, drehbar aneinander befestigt sind.

4. Der Mechanismus nach Forderung 1, bei dem der erste (110) und der zweite Körper (120) eine offene Ringform haben.

5. Exoskelett-Mechanismus nach Forderung 1, der auch drei Freiheitsgrade pro Schultergelenk aufweist, umfassend ein erstes, ein zweites und ein drittes Drehgelenk, von denen mindestens eines ein elektro- oder magnetorheologisches fluidartiges Gelenk nach dem Gelenk-Mechanismus nach Forderung 1 ist, wobei ihre jeweiligen Drehachsen an demselben Punkt konkurrieren, der mit dem Zentrum des Schultergelenks zusammenfällt, wobei diese Drehgelenke durch jeweilige Glieder miteinander verbunden sind (400, 410,420, 430).

6. Ein exoskelettaler Mechanismus nach Forderung 5 enthält auch:
- ein erstes Schulterdrehgelenk (100), um eine Adduktions-Abduktions-Bewegung der Schulter zu realisieren, mit einem ersten Körper (110), der über ein erstes Schultergelenk (400) fest mit dem Torso des Benutzers verbunden ist, und einem zweiten Körper (120), der sich auf dem ersten Körper dreht, insbesondere innerhalb einer Winkelauslenkung von 100°;
- ein zweites Schulterdrehgelenk (100) für die Biege-Extensions-Bewegung der Schulter, mit einem ersten Körper (110), der mit dem zweiten Körper (120) des ersten Drehgelenkes (100) über ein zweites Schulterglied (410) fest verbunden ist, und einem zweiten Körper (120), der sich auf dem ersten Körper (110), insbesondere innerhalb einer Winkelauslenkung von 180°, dreht;
- ein drittes Schulterdrehgelenk (300) für die Innen-Außen-Rotationsbewegung der Schulter, oder medial-laterale Rotation, mit einem ersten Körper (110), der mit dem zweiten Körper (120) des zweiten Schulterdrehgelenk (100) über ein drittes Schulterglied (420) fest verbunden ist, und einem zweiten Körper (120), der sich auf dem ersten Körper (110) insbesondere innerhalb einer Winkelauslenkung von 180° dreht;

7. Exoskelett-Mechanismus nach Forderung 6, bei dem das dritte Drehgelenk (300) der Schulter mit dem ersten (110) und dem zweiten Körper (120) mit offener Ringform hergestellt ist.

8. Exoskelett-Mechanismus nach Forderung 1, der auch zwei Freiheitsgrade für das Ellenbogengelenk offenbart, umfassend zwei Ellenbogendrehgelenk, deren jeweilige Drehachsen in demselben Drehzentrum in der Mitte des Ellenbogengelenks konkurrieren, wobei mindestens eines oder beide der Ellenbogendrehgelenk die Gelenke eines elektro- oder magnetorheologischen Fluidtyps gemäß dem Gelenk des Mechanismus nach Forderung 1 sind, verbunden durch Links (430, 440, 450).

9. Der Mechanismus des Exoskeletts gemäß Anspruch 8 enthält auch:
- ein erstes Ellbogendrehgelenk (100) für die Biege-Streck-Bewegung des Ellbogens, insbesondere mit einem ersten Körper (110), der so konfiguriert ist, dass er über ein erstes Ellbogengelenk (430) am Unterarm (620) befestigt werden kann, und einem zweiten Körper (120), der sich auf dem ersten Körper (110) dreht,
- ein zweites Ellbogendrehgelenk (300), das fixiert ist, um die Pronosupinationsbewegung des Ellbogens zu gewährleisten, mit einem ersten Körper (110), der an dem zweiten Körper (120) des ersten Ellbogendrehgelenkes durch ein zweites Ellbogenglied (440) befestigt ist, und einem zweiten Körper (120), der sich auf dem ersten Körper (110) dreht.

10. Das Exoskelettsystem nach Forderung 9, bei dem das zweite Ellbogendrehgelenk (300) aus dem ersten (110) und dem zweiten Körper (120) mit offener Ringform besteht.

11. Der Exoskelett-Mechanismus nach Forderung 9, bei dem die Flexo-Extensions-Bewegung des ersten (100) Ellenbogendrehmoment innerhalb einer Winkelauslenkung von 140° und die Prono-Supinations-Bewegung des zweiten (300) Ellenbogendrehmoment innerhalb einer Winkelauslenkung von 180° begrenzt ist.

12. Exoskelett-Mechanismus nach Forderung 1, der auch die Eigenschaft aufweist, für eine obere Extremität zu sein und fünf oder mehr Freiheitsgrade zu haben, einschließlich Drehgelenk, von denen mindestens eines ein elektro- oder magnetorheologisches fluidartiges Gelenk nach dem Gelenk-Mechanismus nach Forderung 1 ist, erhalten durch die serielle Kopplung eines Exoskelett-Mechanismus für das Schultergelenk und eines Exoskelett-Mechanismus für das Ellbogengelenk nach Anspruch 5 bzw. 8, wobei der erste Körper (110) des ersten Drehgelenkes (100) des Ellbogens mit dem zweiten Körper (120) des dritten Drehgelenkes der Schulter (300) durch das erste Glied (430) des Ellbogens verbunden ist.

13. Ein Exoskelett-Mechanismus für die obere Extremität nach Forderung 8, bei dem die Prono-Supinationsbewegung durch ein drittes Ellenbogenglied in Form eines Handgriffs (450) ausgeführt wird, das so konfiguriert ist, dass es vom Benutzer gehandhabt werden kann (630), oder Verbandsform (455), konfiguriert für um die Handfläche oder das Handgelenk des Benutzers gewickelt sein, wobei das als drittes Ellbogenglied (450,455) bezeichnete Glied mit dem zweiten Ellbogendrehgelenk verbunden ist, wobei das als drittes Ellbogenglied (450,455) bezeichnete Glied so konfiguriert ist, dass es eine kinematische Kette bildet, die mit dem Glied des Benutzers verbunden ist, und die Übereinstimmung zwischen der Rotation des zweiten Drehgelenkes und der Pronosupinationsbewegung des Handgelenks des Benutzers sicherstellen.

14. Ein Exoskelett-Myoskelett-System (700), das einen Exoskelett-Mechanismus (600) einschließt, gemäß einem der Forderungen 5, 8 oder 12, und die auch eine von der Gruppe ausgewählte Elektrodenanordnung enthält, besteht aus: Schulter-Elektroden (710), Arm-Elektroden (720) und Unterarm-Elektroden (730), konfiguriert für die direkte Platzierung in Kontakt mit der Hautoberfläche des Benutzers, insbesondere mit Hilfe eines elastischen Stütznetzes, wobei die Elektrodenanordnung (710, 720, 730) so konfiguriert ist, dass sie elektrische Impulse an bestimmte periphere Nerven abgibt, um so die Muskelkontraktion und/oder die Bewegung der Körpergelenke zu stimulieren und/oder elektrische Impulse zu erfassen, um das an der Oberfläche entwickelte elektrische Potential zu bestimmen.

15. Ein Exoskelettsystem (800) mit einem Exoskelett-Mechanismus (600) gemäß einem der Ansprüche 5, 8 oder 12, das ein virtuelles oder Gerät für virtuelle oder erweiterte Realität (810) für Anwendungen in der Rehabilitation, Fitness, Unterhaltung usw. integriert, wobei das Gerät für virtuelle oder erweiterte Realität (810) von einer Gruppe ausgewählt wird, besteht aus: einen Helm, ein Visier oder eine andere Vorrichtung, die so konfiguriert ist, dass sie vom Benutzer getragen werden kann, dies Gerät für virtuelle oder erweiterte Realität (810) ist so konfiguriert, dass sie von der genannten Exoskelettvorrichtung Positions-, Geschwindigkeits- und Kraftdaten des Exoskelettmechanismus empfängt und mit dem genannten Benutzer entsprechend den genannten Daten interagiert, insbesondere gibt es ein doppeltes Exoskelett (600) mit Gelenken vom Typ elektro- oder magneto-reologische Flüssigkeit in Kombination mit dem genannten Gerät für virtuelle oder erweiterte Realität. (810).

## Revendications

1. Un mécanisme comprenant au moins deux liaisons (400-410; 410-420; 420-430) reliées par un joint (100; 100; 300), dans lequel ce joint est de type fluide électro- ou magnétorhéologique et comprend:
- un premier corps (110) et un second corps (120) montés en coulisse l' un à l' autre,
- dans lequel le premier corps (110) comprend un piston (130) ayant une tête (131) et une tige (132),
- dans lequel le second corps comprend une chambre (140) dans laquelle le piston (130) est engagé pour coulisser, la chambre (140) formant un circuit fermé rempli d'un fluide électro- ou magnétorhéologique, où ce circuit a une première branche (151) de cette chambre (140) où il coulisse de manière étanche ce piston (130) et une deuxième branche (155) de cette chambre (140) où sont présentes au moins une paire d' électrodes (160) faisant face l' une à l' autre, définissant entre ces électrodes un méat (170) dans lequel ce fluide est présent;
- une unité d'alimentation électrique (200) destiné à alimenter ces électrodes (160) de ce second corps (120) de manière à créer un champ électrique ou magnétique traversant ce méat (170);
- ce piston (130) de ce premier corps (110) étant configuré pour pousser ce fluide dans ce méat (170) et à passer ainsi entre ces électrodes (160) à chaque fois qu' un mouvement relatif se produit entre ce premier (110) et ce second corps (120);
- et cette alimentation électrique (200) étant configuré pour moduler ce champ électrique ou magnétique des électrodes (160) de ce second corps (120) de manière à modifier la résistance au passage de ce fluide dans ce méat (170) lorsque ce mouvement relatif se produit entre ce premier (110) et le deuxième corps (120);
- dans lequel ce premier corps (110) et ce second corps (120) sont montés de façon pivotante l' un autour de l' autre autour d'un centre de rotation (210), et dans lequel ce piston (130) a cette tige (132) de forme curviligne concentrique à ce centre de rotation (210), et dans lequel cette première branche (151) de cette chambre (140) et de cette deuxième branche (155) de cette chambre (140) présentent une forme curviligne concentrique à ce centre de rotation (210).

2. Le mécanisme selon la revendication 1, dans lequel cette paire d'électrodes (160) de ce deuxième corps (120) est constitué d'une première électrode et d'une seconde électrode ayant chacune plusieurs saillies et retours parallèles et concentriques sur cet axe et s' étendant autour de la circonférence pour un angle prédéterminé, les deux électrodes s' intercalant l' un de l' autre, de sorte que chaque saillie circonférentielle de la première électrode s'interpose avec les saillies circonférentielles adjacentes de la deuxième électrode.

3. Le mécanisme selon la revendication 1, dans lequel le premier corps (110) et le second corps (120) sont montés de manière pivotante l' un à l' autre au moyen de roulements (261), en particulier de roulements, incorporés dans des sièges (260) découpés dans le premier corps (110) et dans le second corps (120), qui réalisent une rotation à faible friction entre le second corps (120) et le premier corps (110).

4. Le mécanisme selon la revendication 1, dans lequel ce premier (110) et deuxième corps (120) ont une forme annulaire ouverte.

5. Un mécanisme exosquelettique selon la revendication 1, qui révèle en outre trois degrés de liberté par articulation de l'épaule, comprenant une première, une deuxième et une troisième paire rotoïdale, dont au moins une est un joint de type fluide électro-électro- soit magnétorhéologique selon le joint du mécanisme de la revendication 1, avec leurs axes de rotation concurrents respectifs en un même point coïncidant avec le centre de l' articulation de l'épaule, où ces couples rotoïdaux sont reliés entre eux par leurs liens respectifs (400, 410, 420, 430).

6. Un mécanisme exosquelettique selon la revendication 5, qui comprend également:
- une première paire rotoïde (100) d'épaule, afin de réaliser un mouvement d'adduction-abduction de l'épaule, ayant un premier corps (110) attaché au corps de l'utilisateur par une première liaison d'épaule (400), et un second corps (120) tournant sur le premier corps, en particulier dans une excursion angulaire de 100°;
- une seconde paire rotoïde (100) d'épaule, pour le mouvement de flexion-extension de l'épaule, ayant un premier corps (110) solidaire du second corps (120) de la première paire rotoïde (100) par l'intermédiaire d'une seconde liaison d'épaule (410), et un second corps (120) tournant sur le premier corps (110), en particulier dans une excursion angulaire de 180°;
- une troisième paire rotoïde (300) d'épaule, pour le mouvement de rotation interne-externe de l'épaule, ou rotation médio-latérale, comportant un premier corps (110) solidaire du deuxième corps (120) de la deuxième paire rotoïde (100) d'épaule par l'intermédiaire d'une troisième liaison d'épaule (420), et un deuxième corps (120) tournant sur le premier corps (110), notamment dans le cadre d'une excursion angulaire de 180°,

7. Un mécanisme exosquelettique selon la revendication 6, dans lequel le troisième couple rotoïdal (300) d' épaule est réalisé avec ce premier (110) et ce second corps (120) avec forme annulaire ouverte.

8. Un mécanisme exosquelettique selon la revendication 1, qui révèle en outre deux degrés de liberté pour l'articulation du coude, comprenant deux couples rotoïdaux de coude avec leurs axes de rotation concurrents respectifs dans un même centre de rotation au centre de l'articulation du coude, dont au moins un de ces couples rotoïdaux de coude sont des joints de type fluide électro soit magnétorhéologique selon le joint du mécanisme de la revendication 1, reliés les uns aux autres par des liaisons (430, 440, 450).

9. Le mécanisme exosquelettique selon la revendication 8 comprend également:
- une première paire rotoïde (100) de coudes, pour le mouvement de flexion-extension du coude, ayant notamment un premier corps (110) configuré pour être fixé à l'avant-bras (620) par une première liaison coudée (430), et un second corps (120) tournant sur le premier corps (110),
- un second couple rotoïdal (300) de coude, fixé pour assurer le mouvement de pronosupination du coude, ayant un premier corps (110) solidaire du second corps (120) du premier couple rotoïdal de coude par une seconde liaison (440) de coude, et un second corps (120) tournant sur le premier corps (110).

10. Le mécanisme exosquelettique selon la revendication 9, dans lequel le second couple rotoïdal (300) de coude est réalisé avec ce premier (110) et ce second corps (120) avec une forme annulaire ouverte.

11. Le mécanisme exosquelettique selon la revendication 9, dans lequel le mouvement de flexion-extension de la première paire rotoïde (100) du coude est délimité dans une excursion angulaire de 140° et le mouvement de pronosupination de la deuxième paire rotoïde (300) du coude est délimité dans une excursion angulaire de 180°.

12. Un mécanisme exosquelettique selon la revendication 1, qui révèle en outre la caractéristique d'être pour un membre supérieur et avoir cinq degrés ou plus de liberté, comprenant les couples rotoïdaux, dont au moins un est un joint de type fluide électro- ou magnétorhéologique selon le joint du mécanisme de la revendication 1, obtenu par accouplement en série d'un mécanisme exosquelettique pour l'articulation de l'épaule et d'un mécanisme exosquelettique pour l'articulation du coude selon les revendications 5 et 8 respectivement, avec le premier corps (110) du premier couple rotoïdal (100) en coude solidaire du deuxième corps (120) du troisième couple rotoïdal d' épaule (300) par le premier maillon (430) de coude.

13. Un mécanisme exosquelettique pour le membre supérieur selon la revendication 8, dans lequel le mouvement de pronostic-supination est réalisé grâce à un troisième lien de coude, en forme de poignée (450), configuré pour être saisi (630) par l'utilisateur, ou en forme de bande (455), configuré pour être enroulé autour de la paume de la main ou du poignet de l'utilisateur, appelé troisième maillon du coude (450,455) étant connecté à la deuxième paire de coude rotoïdale, appelé troisième maillon du coude (450,455) étant configuré pour établir une chaîne cinématique qui est verrouillée au membre de l'utilisateur et assurer la correspondance entre la rotation de la deuxième paire de coudes rotoïdes et le mouvement de pronosupination de l'articulation du poignet de l'utilisateur.

14. Un système exosquelettique-myoélectrique (700) comprenant un mécanisme exosquelette (600), selon l'une des revendications 5, 8 ou 12, et comprenant en outre un réseau d'électrodes sélectionnées dans le groupe consistant en: des électrodes d'épaule (710), des électrodes de bras (720) et des électrodes d'avant-bras (730), configurées pour être placées directement en contact avec la surface de la peau de l'utilisateur, en particulier au moyen d'un filet de support élastique, appelé réseau d'électrodes (710, 720, 730) étant configuré pour fournir des impulsions électriques à des nerfs périphériques spécifiques, de manière à stimuler la contraction musculaire et/ou le mouvement des articulations du corps et/ou à détecter les impulsions électriques pour déterminer le potentiel électrique de surface développé par les districts musculaires liés auxdites surfaces de la peau.

15. Un système exosquelettique (800) comprenant un mécanisme exosquelettique (600), selon l'une des revendications 5, 8 ou 12, intégrant un dispositif de réalité virtuelle ou augmentée (810) pour des applications dans le domaine de la réhabilitation, du fitness, du divertissement, etc., ce dispositif de réalité virtuelle ou augmentée (810) étant choisi par un groupe composé : un casque, une visière ou un autre dispositif configuré pour être porté par l'utilisateur, ce dispositif de réalité virtuelle ou augmentée (810) étant configuré pour recevoir des données de position, de vitesse et de force du mécanisme exosquelettique de ce dispositif et interagir avec cet utilisateur en fonction de ces données, en particulier, il existe un double exosquelette (600) constitué de joints de type fluide électro- ou magnéto- rhéologique, en combinaison avec ce dispositif de réalité virtuelle ou augmentée (810).
